# EUROPEAN PATENT APPLICATION

(11) **EP 4 487 872 A2**
(43) Date of publication of application: **08.01.2025**
(21) Application number: 24215210.6
(22) Date of filing: 13.04.2021
(51) Int. Cl.: A61K 47/44

(54) **RESINIFERATOXIN COMPOSITIONS**

(30) Priority: 15.04.2020 EP 20169668
(62) Divisional of application: 21717110.7
(71) Applicant: Grünenthal GmbH, 52078 Aachen (DE)
(72) Inventor: BERNKOP-SCHNÜRCH, Andreas, 6080 Igls (AT); VALDENAIRE, Olivier, 4059 Basel (CH); GILLER, Thomas, 4451 Wintersingen (CH)
(74) Representative: Kutzenberger Wolff & Partner

(57) **Abstract**

The invention provides non-aqueous solid and liquid compositions comprising resiniferatoxin and a surfactant. The non-aqueous solid and liquid compositions may be used to prepare aqueous compositions that are used in the treatment of pain, specifically osteoarthritis-related joint pain. Moreover, the invention provides kits containing the non-aqueous solid and liquid compositions and a diluent and methods of preparing the compositions. The non-aqueous solid and liquid compositions allow for longer storage and recovery of resiniferatoxin compared to resiniferatoxin alone prior to reconstitution with a diluent.

## Description

### FIELD OF THE INVENTION

The present invention relates to the field of pharmaceutics, and concerns compositions of resiniferatoxin, their manufacture and their use.

### BACKGROUND OF THE INVENTION

Resiniferatoxin (RTX) is a naturally occurring compound produced by the Moroccan cactus *Euphorbia resinifera* and is a highly potent analog of capsaicin, the active ingredient of the red chili pepper. At very small dosages, resiniferatoxin is known as a transient receptor potential cation channel subfamily V member 1 (TRPV1) agonist, and has been investigated for therapeutic efficacy in a variety of diseases or ailments such as pain relief, overactive bladder, interstitial cystitis, and rhinitis.

Resiniferatoxin is known to easily bind to a variety of substances and surfaces, making handling of very extremely small quantities required for low dosages of the compound challenging. Moreover, when thawed it decomposes into fragments and therefore is thawed immediately before use and diluted in a solvent such as ethanol or dimethyl sulfoxide to prevent decomposition.

It is thus an object of the present invention to provide compositions of resiniferatoxin that may be stored for longer periods of time without significant decomposition, in particular non-aqueous solid and liquid compositions of resiniferatoxin and a surfactant with optionally other components. The non-aqueous compositions may be diluted with a diluent such as an aqueous diluent for use in therapeutic applications. Kits, methods of manufacture, and methods of use of the resiniferatoxin compositions are also provided. Further objects of the invention will be clear on the basis of the following description of the invention, examples and clauses.

### SUMMARY OF THE INVENTION

In a first aspect, the invention relates to non-aqueous solid or non-aqueous liquid compositions of resiniferatoxin comprising (a) resiniferatoxin; and (b) a surfactant. In certain embodiments, the non-aqueous solid compositions are lyophilized solids. The invention also relates to kits comprising (a) a first kit component comprising the non-aqueous solid or non-aqueous liquid composition; and (b) a second kit component comprising a diluent. Methods of using the non-aqueous solid and non-aqueous liquid compositions for the manufacture of a medicament for the treatment of pain are also contemplated. In a specific embodiment, the pain is osteoarthritis-related joint pain.

In a further aspect, the present invention provides aqueous compositions of resiniferatoxin comprising (a) from about 0.005 µM to about 10 µM resiniferatoxin; (b) from about 0.01 vol % to about 10 vol % of a surfactant; and (c) at least one buffering agent; wherein the composition has a pH from about 7.7 to about 8.3. The aqueous compositions may be used for the treatment of pain. In some embodiments, the invention provides methods of treating pain, comprising administering to a subject a therapeutically effective amount of the aqueous compositions disclosed herein. In some embodiments, the aqueous compositions are adapted for intra-articular administration. In a specific embodiment, the pain is osteoarthritis-related joint pain.

### DETAILED DESCRIPTION OF THE INVENTION

### Definitions

The term "about" or the like in connection with an attribute or value includes the exact attribute or precise value, as well as any attribute or value typically considered to fall within the normal or accepted variability associated with the technical field, and methods of measuring or determining said attribute or value. The term "about" refers usually to a range of values ± 10% of a specified value. For example, the phrase "about 200" includes ± 10% of 200, or from 180 to 220.

The term "kit" as used herein means that the components comprised in said kit are provided physically separable and distinguishable from one another as different components but are provided or sold together for the purpose of being administered, or used, together.

The terms "lyophilized", "lyophilization", and "lyophilize" as used herein refer to a process of freeze-drying, where a sample is first frozen and dried, typically under a vacuum, to remove water and/or other solvents.

The term "non-aqueous" as used herein refers to a composition, either solid or liquid, that is substantially free of water (other than water of hydration), preferably less than 2 percent by weight of water as measured by standard techniques such as, for example, a thermogravimetric measurement. The non-aqueous compositions described herein may contain small amounts of water absorbed from ambient moisture.

The terms "resiniferatoxin" and "RTX" as used herein refer to a naturally occurring chemical produced by the Moroccan cactus *Euphorbia resinifera* or synthetically produced and has the following structure and a molar weight of 628.71:

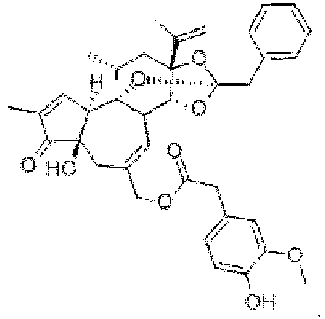

The term "D-α-tocopherol polyethylene glycol succinate" is synonymously used herein with the term "D-α-Tocopherol polyethylene glycol 1000 succinate" and the abbreviation "TPGS". D-α-tocopherol polyethylene glycol succinate is a non-ionic surfactant.

The term "solid composition" as used herein refers to solids in crystalline, powder, and/or amorphous form, or any mixtures thereof. It is in the solid state at normal temperature and pressure (NTP) as defined by the US National Institute of Standards and Technology (NIST). The solid compositions described herein may contain (in addition to any water of hydration) trace amounts of water or other solvents, preferably less than 2 wt % of the water or other solvents. The solid composition may be stored at room temperature or below, preferably about 4 °C, about 10 °C, or about 25 °C prior to reconstitution with a liquid solvent.

The term "buffer" or "buffer solution" means a combination of two or more constituents which buffer (i.e. minimize the change of) the pH value upon addition of an acid or base. Buffers or buffer solutions may further comprise auxiliary compounds, such as neutral salts like sodium chloride that contribute little to the buffer effect, but which are incorporated for physiological reasons, e.g. to adjust the osmolality of a buffer solution.

### Solid Compositions

In one aspect, the invention provides non-aqueous solid compositions of resiniferatoxin comprising resiniferatoxin and a surfactant. Optionally, the compositions comprise one or more other additives. In some of the preferred embodiments, the solid composition is a lyophilized composition, also referred to as a lyophilate or lyophilizate. In some embodiments, the surfactant may be a non-ionic surfactant. Exemplary non-ionic surfactants include D-α-tocopherol polyethylene glycol succinate, PEG-20 hydrogenated castor oil, PEG-35 hydrogenated castor oil, PEG-40 hydrogenated castor oil, PEG-60 hydrogenated castor oil, PEG-100 hydrogenated castor oil, PEG-1000 hydrogenated castor oil, PEG-2000 hydrogenated castor oil, PEG-4000 hydrogenated castor oil, PEG-6000 hydrogenated castor oil, polysorbate 20, polysorbate 40, polysorbate 60, polysorbate 80, and polysorbate 100. In some embodiments, the surfactant is selected from the group consisting of D-α-tocopherol polyethylene glycol succinate, PEG-60 hydrogenated castor oil, PEG-40 hydrogenated castor oil, PEG-35 hydrogenated castor oil, and polysorbate 80. In a particular embodiment, the surfactant is D-α-tocopherol polyethylene glycol succinate or polysorbate 80. In a more particular embodiment, the surfactant is D-α-tocopherol polyethylene glycol succinate.

The compositions may further include a polyethylene glycol and/or a further surfactant, where the further surfactant is optionally a poloxamer. Exemplary polyethylene glycols include PEG 1000, PEG 1450, PEG 1600, PEG 3000, PEG 3350, PEG 4000, PEG 4500, PEG 6000, PEG 8000, and PEG 20000. In some embodiments, the polyethylene glycol is PEG 6000. The poloxamer may, for example, be poloxamer 101, 182, 188, 237, 331, 338, or 407. In some embodiments, the poloxamer is poloxamer 188, 237 or 407. In another embodiment, the poloxamer is poloxamer 188.

Other optional additives include solid components of a buffer solution. The components may include, for example, NaCl, CaCl₂ x 2 H₂O, KCl, *tris*(hydroxymethyl)aminomethane, and combinations thereof. In a particular embodiment, the non-aqueous solid composition comprises resiniferatoxin, D-α-tocopherol polyethylene glycol succinate, NaCl, CaCl₂ x 2 H₂O, KCl, and tris(hydroxymethyl)aminomethane and is preferably lyophilized. In some embodiments, the non-aqueous solid compositions disclosed herein do not comprise a monosaccharide or sugar alcohol.

The non-aqueous solid compositions of resiniferatoxin may be obtained by combining resiniferatoxin, a surfactant, and the optional additives, if any, in a solvent to form a solution or dispersion, then drying the solution or dispersion. For example, a stock solution of resiniferatoxin in an organic solvent such as absolute ethanol in a concentration of about 0.1 to about 1000 µg/mL, about 0.2 to about 200 µg/mL, or about 20 to about 50 µg/mL may be prepared. Other organic solvents that may be used include glycerin or DMSO. The stock solution (e.g., 50 µL) may be diluted with a diluent (e.g., 950 µL) containing the surfactant and optionally polyethylene glycol or a further surfactant (e.g., a poloxamer) followed by drying. In some embodiments, the diluent is an aqueous diluent. In some embodiments, the aqueous diluent comprises at least one buffering agent and optionally at least one isotonizing agent. In some embodiments, the at least one buffering agent is adapted to maintain a pH from about 7.7 to about 8.3. In other embodiments, the aqueous diluent comprises about 140 - 150 mM NaCl, about 3 - 5 mM CaCl₂ x 2 H₂O, about 3 - 5 mM KCl, about 8 - 12 mM *tris*(hydroxymethyl)aminomethane, and has a pH from about 7.7 to about 8.3. In a particular embodiment, the aqueous diluent comprises about 147 mM NaCl, about 4 mM CaCl₂ x 2 H₂O, about 4 mM KCl, about 10 mM *tris*(hydroxymethyl)aminomethane, and has a pH of about 8.0.

The drying or solvent removal may be carried out by a variety of techniques including vacuum evaporation, spray drying, micro encapsulation, lyophilization, other drying techniques, or combinations thereof. In some embodiments, the solution is frozen and dried by lyophilization.

The non-aqueous solid compositions are typically more stable at or below room temperature than solid resiniferatoxin alone or aqueous liquid compositions of resiniferatoxin. The solids may be stored at or below room temperature before reconstitution and use. Exemplary storage temperatures include at about 0 °C, about 4 °C, or about 25 °C or within the ranges of about -5 °C to about 25 °C, about 0 °C to about 25 °C, about 0 °C to about 15 °C, or about 0 °C to about 10 °C, or about 0 °C to about 5 °C.

In some further embodiments, the non-aqueous solid compositions of the invention are characterized in that they are readily dissolvable in an aqueous carrier or diluent. In these embodiments, the solid composition may be combined with a preferably sterile aqueous diluent such that upon shaking a liquid solution is obtained which contains the resiniferatoxin in dissolved form. In some embodiments, at least 90 % of the resiniferatoxin is recovered after dissolution of the solid in an aqueous diluent. In other embodiments, at least 95 % of the resiniferatoxin is recovered after dissolution of the solid in an aqueous diluent. In some embodiments, at least 90% of the resiniferatoxin is recovered after storage at about -5 °C to about 25 °C, about 0 °C to about 25 °C, about 0 °C to about 15 °C, or about 0 °C to about 10 °C, or about 0 °C to about 5 °C for up to about 1 to about 24 months, about 6 to about 24 months, or about 12 to about 24 months. In a particular embodiment, at least 90% of the resiniferatoxin is recovered after storage at about 0 °C to about 25 °C for up to about 3 to about 24 months. In another particular embodiment, at least 95% of the resiniferatoxin is recovered after storage at about 0 °C to about 25 °C for up to about 3 to about 12 months.

### Liquid Compositions

In another aspect, the invention provides non-aqueous liquid compositions of resiniferatoxin comprising resiniferatoxin and a surfactant. Optionally, the compositions comprise one or more other additives. Like the non-aqueous solid compositions described herein, the non-aqueous liquid compositions provide stable formulations of resiniferatoxin that may be readily converted to liquid aqueous compositions that may be used e.g. as solutions for injection for delivering effective amounts of resiniferatoxin to patients in need thereof. The non-aqueous liquid compositions of the invention may also be referred to as concentrates or pre-concentrates, respectively.

The non-aqueous liquid compositions of resiniferatoxin may be obtained by combining resiniferatoxin, a surfactant, and optionally other additives in a solvent to form a non-aqueous solution. In some embodiments, the solvent is absolute ethanol. In some embodiments, the surfactant may be a non-ionic surfactant. In some embodiments, the non-aqueous liquid compositions comprise (a) from about 0.005 µM to about 10 µM resiniferatoxin, preferably from about 0.01 µM to about 1 µM resiniferatoxin, more preferably from about 0.1 µM to about 1 µM resiniferatoxin, even more preferably from about 0.1 µM to about 0.2 µM resiniferatoxin. Exemplary non-ionic surfactants include D-α-tocopherol polyethylene glycol succinate, PEG-20 hydrogenated castor oil, PEG-35 hydrogenated castor oil, PEG-40 hydrogenated castor oil, PEG-60 hydrogenated castor oil, PEG-100 hydrogenated castor oil, PEG-1000 hydrogenated castor oil, PEG-2000 hydrogenated castor oil, PEG-4000 hydrogenated castor oil, PEG-6000 hydrogenated castor oil, polysorbate 20, polysorbate 40, polysorbate 60, polysorbate 80, and polysorbate 100. In some embodiments, the surfactant is selected from the group consisting of D-α-tocopherol polyethylene glycol succinate, PEG-60 hydrogenated castor oil, PEG-40 hydrogenated castor oil, PEG-35 hydrogenated castor oil, and polysorbate 80. In certain embodiments, the surfactant is D-α-tocopherol polyethylene glycol succinate. In other embodiments, the surfactant is polysorbate 80. The optional additives may include a polyethylene glycol and/or a further surfactant, where the further surfactant is optionally a poloxamer. Exemplary polyethylene glycols include PEG 1000, PEG 1450, PEG 1600, PEG 3000, PEG 3350, PEG 4000, PEG 4500, PEG 6000, PEG 8000, and PEG 20000. In some embodiments, the polyethylene glycol is PEG 6000. The poloxamer may, for example, be poloxamer 101, 182, 188, 237, 331, 338, or 407. In some embodiments, the poloxamer is poloxamer 188, 237 or 407. In another embodiment, the poloxamer is poloxamer 188.

In a particular embodiment, the non-aqueous liquid composition comprises resiniferatoxin and polysorbate 80. In another particular embodiment, the non-aqueous liquid composition comprises resiniferatoxin and D-α-tocopherol polyethylene glycol succinate. In some embodiments, the non-aqueous liquid compositions disclosed herein do not comprise a monosaccharide or sugar alcohol.

The non-aqueous liquid compositions of resiniferatoxin may be obtained by combining resiniferatoxin, a surfactant, and the optional additives in a solvent to form the non-aqueous liquid composition e.g. a aseptic solution with the desired concentrations of RTX and the corresponding surfactant and/or poloxamer in EtOH abs. is prepared and an aliquot of this solution is distributed into vials and sealed with stoppers and crimped.

The inventors have found that the non-aqueous liquid compositions are more stable at or below room temperature than solid resiniferatoxin alone or aqueous liquid compositions of resiniferatoxin. Exemplary storage temperatures include at about 0 °C, about 4 °C, or about 25 °C or within the ranges of about -5 °C to about 25 °C, about 0 °C to about 25 °C, about 0 °C to about 15 °C, or about 0 °C to about 10 °C, or about 0 °C to about 5 °C. In some embodiments, at least 90% of the resiniferatoxin is recovered after storage at about -5 °C to about 25 °C, about 0 °C to about 25 °C, about 0 °C to about 15 °C, or about 0 °C to about 10 °C, or about 0 °C to about 5 °C for up to about 1 to about 24 months, about 6 to about 24 months, or about 12 to about 24 months. In a particular embodiment at least 90% of the resiniferatoxin is recovered after storage at about 0 °C to about 25 °C for up to about 3 to about 24 months. In another particular embodiment at least 95% of the resiniferatoxin is recovered after storage at about 0 °C to about 25 °C for up to about 3 to about 12 months. *Kits*

In another aspect, the invention provides a kit comprising (a) a first kit component comprising a non-aqueous solid composition or a non-aqueous liquid composition as described herein and (b) a second kit component comprising a diluent.

In some embodiments, the diluent may be an aqueous solution or an organic solvent. Exemplary organic solvents include absolute ethanol, glycerin, and dimethyl sulfoxide (DMSO). A particular diluent is an aqueous diluent. The aqueous diluent may comprise only water or may comprise at least one buffering agent and optionally at least one isotonizing agent. The buffering agent may be adapted to maintain a pH from about 7.7 to about 8.3 upon combining the first and second kit components. In some embodiments, the aqueous diluent comprises about 140 - 150 mM NaCl, about 3 - 5 mM CaCl₂ x 2 H₂O, about 3 - 5 mM KCl, about 8 - 12 mM *tris*(hydroxymethyl)aminomethane, and has a pH from about 7.7 to about 8.3. In a particular embodiment, the aqueous diluent comprises about 147 mM NaCl, about 4 mM CaCl₂ x 2 H₂O, about 4 mM KCl, about 10 mM *tris*(hydroxymethyl)aminomethane, and has a pH of about 8.0.

In some embodiments, the first kit component is a non-aqueous solid composition comprising resiniferatoxin, a surfactant, and optionally at least one buffering agent and optionally at least one isotonizing agent, and the second kit component is water. In certain embodiments, the non-aqueous solid composition is lyophilized. In some embodiments, the buffering agent and optional isotonizing agent comprise NaCl, CaCl₂ x 2 H₂O, KCl, and *tris*(hydroxymethyl)-aminomethane. In certain embodiments, when the first kit component (non-aqueous solid composition) is combined with the second kit component (water), the resulting aqueous solution has a pH from about 7.7 to about 8.3.

In other embodiments, the first kit component is a non-aqueous liquid composition comprising resiniferatoxin and a surfactant, and the second kit component is an aqueous diluent comprising at least one buffering agent and optionally at least one isotonizing agent. In some embodiments, when the first kit component (non-aqueous liquid composition) is combined with the second kit component (aqueous diluent containing at least one buffering agent and optionally at least one isotonizing agent), the resulting aqueous solution has a pH from about 7.7 to about 8.3.

In some embodiments, the kit may be supplied in the form of:
(a) separate compartments of one primary package (such as a sachet divided into two or more 'sub-pouches' by a laminating seam, or a glass vial filled with one kit component and the other kit component being held in the screw-top lid of said glass vial);
(b) separate primary packages packaged together within one secondary package (such as separate sets of sachets or glass vials for two or more kit components, the two or more sachet-sets or glass vial-sets being sold in one and the same folded box);
(c) separate primary packages packaged in two or more separate secondary packages which are in turn held together by paper or plastic wrappers, ribbons, sleeves or the like (such as separate sets of sachets or glass vials for two or more kit components, the two or more sachet-sets or glass vial-sets being sold in two or more cardboard boxes, the latter being wrapped with a shrink foil wrapper); or
(d) combinations thereof (such as a first kit-component being provided in multiple-dose cardboard drum, optionally with a dosing spoon, the cardboard drum being sold in a folded box together with a multitude of glass vials, each glass vial containing a second kit-component).

Optionally, the kits of the invention may further comprise written instructions on how to best, or preferably, combine and use the two or more kit components.

### Aqueous compositions for injection and use

In another aspect, the invention provides aqueous compositions of resiniferatoxin comprising (a) resiniferatoxin (b) a surfactant (c) and at least one buffering agent, wherein the composition has a pH from about 7.7 to about 8.3. The aqueous compositions may be prepared by combining the first and second kit components as described above. In some embodiments, the aqueous compositions comprise (a) from about 0.005 µM to about 10 µM resiniferatoxin, preferably from about 0.01 µM to about 1 µM resiniferatoxin, more preferably from about 0.1 µM to about 1 µM resiniferatoxin, even more preferably from about 0.1 µM to about 0.2 µM resiniferatoxin; (b) from about 0.01 vol % to about 10 vol % of a surfactant, preferably from about 0.01 vol % to about 1 vol % of a surfactant, more preferably from about 0.1 vol % to about 1 vol % of a surfactant, even more preferably about 0.2 vol % to about 0.5 vol % of a surfactant, in particular 0.35 vol % of a surfactant; and (c) at least one buffering agent; wherein the composition has a pH from about 7.7 to about 8.3. In other embodiments, the aqueous compositions comprise (a) from about 0.005 µM to about 10 µM resiniferatoxin, preferably from about 0.01 µM to about 1 µM resiniferatoxin, more preferably from about 0.1 µM to about 1 µM resiniferatoxin, even more preferably from about 0.1 µM to about 0.2 µM resiniferatoxin; (b) from about 0.01 vol % to about 10 vol % of a surfactant, preferably from about 0.01 vol % to about 1 vol % of a surfactant, more preferably from about 0.1 vol % to about 1 vol % of a surfactant, even more preferably about 0.2 vol % to about 0.5 vol % of a surfactant, in particular 0.35 vol % of a surfactant; (c) about 140 - 150 mM NaCl; (d) about 3 - 5 mM CaCl₂ x 2 H₂O; (e) about 3 - 5 mM KCl; (f) and about 8 - 12 mM *tris*(hydroxymethyl)aminomethane; wherein the composition has a pH from about 7.7 to about 8.3. In particular embodiments, the aqueous compositions comprise (a) from about 0.01 µM to about 1 µM resiniferatoxin, preferably from about 0.1 µM to about 1 µM resiniferatoxin, more preferably from about 0.1 µM to about 0.2 µM resiniferatoxin; (b) from about 0.01 vol % to about 1 vol % of a surfactant, more preferably from about 0.1 vol % to about 1 vol % of a surfactant, even more preferably about 0.2 vol % to about 0.5 vol % of a surfactant, in particular 0.35 vol % of a surfactant0.35 vol % of a surfactant; (c) about 147 mM NaCl; (d) about 4 mM CaCl₂ x 2 H₂O; (e) about 4 mM KCl; (f) and about 10 mM *tris*(hydroxymethyl)aminomethane; wherein the composition has a pH of about 8.0. In particular embodiments, the aqueous compositions do not comprise a monosaccharide or sugar alcohol.

### Methods of Use

The non-aqueous solid compositions, the non-aqueous liquid compositions and/or the aqueous composition of resiniferatoxin disclosed herein may be used for the manufacture of a medicament for the treatment of pain. Thus the present invention provides the non-aqueous solid compositions, the non-aqueous liquid compositions and/or the aqueous compositions of resiniferatoxin disclosed herein for use as a medicament. In some embodiments, the compositions disclosed herein are used for the treatment of pain. Thus the present invention provides the non-aqueous solid compositions, the non-aqueous liquid compositions and/or the aqueous compositions of resiniferatoxin disclosed herein for use in a method for the treatment of pain. Also provided are the non-aqueous solid compositions , the non-aqueous liquid compositions and/or the aqueous compositions of resiniferatoxin disclosed herein for the manufacture of a medicament for the treatment of pain. In other embodiments, the disclosure provides methods for treating pain, comprising administering to a subject a therapeutically effective amount of any of the non-aqueous solid compositions, the non-aqueous liquid compositions and/or the aqueous compositions described herein. In particular embodiments, the aqueous compositions are adapted for intra-articular administration. In particular embodiments, the pain is osteoarthritis-related joint pain.

The following examples serve to illustrate the invention, however should not be understood as restricting the scope of the invention.

### EXAMPLES

### Materials

The following materials were used to prepare exemplary non-aqueous solid and liquid compositions described herein: acetonitrile (Lot: STBH6791, Sigma-Aldrich, Austria); ammonium formate (Lot: BCBD3147V, Sigma-Aldrich, Austria); calcium chloride dihydrate (Lot: S28238-156, Sigma-Aldrich, Austria); Cremophor EL (Lot: 86741436W0, BASF; Germany); Cremophor RH60 (Lot: 91191656P0, BASF; Germany); formic acid (Lot: 396236476, Carl Roth, Karlsruhe, Germany); hydrochloric acid (Lot: H2116K1, Carl Roth, Germany); mannitol (Lot: 1560453, Thermo Scientific, Austria); ortho - phosphoric acid 85% for HPLC (Lot: Z0440928, Merck, Germany); PEG6000 (polyethylene glycol 6000; Lot: 2005TS1586, Croda; Germany); potassium chloride (Lot: 351173748, Carl Roth, Germany); RTX solution 10 mg·mL⁻¹ provided by Mestex (Lot: LIMS200949145, Carbogen Amcis, Switzerland); sodium chloride (Lot: 177255242, Carl Roth, Germany); sodium phosphate monobasic monohydrate (Lot: BCBG8983V, Sigma-Aldrich, Austria); TPGS (D-α-Tocopherol polyethylene glycol 1000 succinate ; Lot: BCBX8795, Sigma-Aldrich, Austria); tris (*tris*(hydroxymethyl)aminomethane, Trizema Ph. Eur, (Lot: S28238-156, Sigma-Aldrich, Vienna, Austria); and Tween 80 (polysorbate 80, Lot: K47176961 602, Merck Millipore, Vienna, Austria).

### Example 1:

### Preparation of non-aqueous solid RTX compositions

A stock solution of 1 mg/ml RTX in EtOH abs. was prepared. From this stock solution, working solutions were prepared containing the desired RTX concentration in EtOH. Test vials were prepared by combining 50 µl of RTX working solution with 950 µl of Buffer containing the corresponding surfactants and/or poloxamers. All prepared vials were homogenized, covered with Para-film, frozen and subsequently lyophilized to produce the non-aqueous solid RTX compositions.

### Exemplary Compositions

Exemplary non-aqueous solid compositions that have been prepared are summarized in Table 1 below.

**Table 1:**

| | Lyophilized RTX/ethanol/Tween/ PEG6000/Mestex buffer | Lyophilized RTX/ethanol/TPGS/ Mestex buffer | Lyophilized RTX/ethanol/TPGS/ PEG6000/Mestex buffer |
|---|---|---|---|
| Composition | 3.2 µg/vial RTX | 3.2 µg/vial RTX | 3.2 µg/vial RTX |
| | 70 mg/vial Tween PEG6000 | 70 mg/vial TPGS | 70 mg/vial TPGS PEG6000 |
| | 1x Mestex buffer | 1x Mestex buffer | 1x Mestex buffer |
| Condition | Lyophilized | Lyophilized | Lyophilized |
| Preparation of final formulation | Reconstitution with water for injection | Reconstitution with water for injection | Reconstitution with water for injection |
| Storage conditions | optional at 4°C and 25°C/60% RH glass vials | 4°C and 25°C/60% RH glass vials | 4°C and 25°C/60% RH glass vials |
| Sampling | optional 1 m, 2 m, 3 m, 6 m, 9 m and 12 m | 1 m, 2 m and 3 m optional 6 m, 9 m and 12 m | 1 m, 2 m and 3 m optional 6 m, 9 m and 12 m |

Table 1 shows non-aqueous solid compositions prepared. "Mestex buffer" (MB) contains 147 mM NaCl, 4 mM CaCl₂ x 2 H₂O, 4 mM KCl, 10 mM *tris*(hydroxymethyl)aminomethane, and has a pH of about 8.0.

### Stability Tests

For certain exemplary non-aqueous compositions described herein, the % values of the amount of resiniferatoxin detected by HPLC after lyophilization and reconstitution are provided in Table 2, below. After the lyophilization process, all vials were sealed and stored at the corresponding conditions as shown in Table 1. At each time point (month (m)), the required number of vials were reconstituted with 1 mL of water for injection by gently shaking the vials until complete dissolution of the lyophilized product was achieved. After 5 min, samples were taken, diluted 1:1 with ethanol and analyzed by HPLC. Moreover, a calibration curve was established at each time point using the RTX dilutions made from the stock solution in order to ensure comparability of the obtained data.

**Table 2:**

| | TPGS/MB | | TPGS/PEG/MB | | Tween/PEG/MB | | Creph. 40/MB | | Tween/MB | | | Creph. E/Mannitol | |
|---|---|---|---|---|---|---|---|---|---|---|---|---|---|
| | 4°C | 25°C | 4°C | 25°C | 4°C | 25°C | 4°C | 25°C | 4°C | 40°C | 40°C | 4°C | 25°C |
| T0 | | | | | | | | | | 101.0 | | | 102.0 |
| 1 m | 94.2 | 92.8 | 96.2 | 92.3 | 100.3 | 94.2 | | | 97.2 | 81.0 | 56.8 | | |
| 2 m | 96.5 | 94.2 | 93.5 | 88.9 | 99.5 | 90.9 | | | 89.5 | 57.0 | 52.6 | | |
| 3 m | 97.9 | 95.9 | 90.3 | 91.3 | | 87.7 | | 93.0 | 86.2 | 55.0 | 60.5 | | |
| 6 m | 97.9 | 91.4 | | | 88.1 | 88.0 | | | | | | | |
| 9 m | 93.3 | 88.0 | | | 85.1 | 88.0 | | | | | | | |
| 12 m | 87.6 | 83.7 | | | 85.3 | n.d. | | | | | | | |

Table 2 shows stability data of non-aqueous solids over 12 months. All reconstituted solutions were clear and colorless. The pH was 8.0 ± 0.2.

### Example 2:

### Preparation of non-aqueous liquid RTX compositions

A aseptic solution with the desired concentrations of RTX and the corresponding surfactant and/or poloxamers in EtOH abs. was prepared. 0.5ml of this solution is distributed into 10R vials and sealed with stoppers and crimped. Such preparations are stored at 5°C.

Exemplary non-aqueous liquid compositions that have been prepared are summarized in Table 3 below.

In addition to the non-aqueous solid compositions RTX was also formulated in EtOH (abs) together with different surfactants as liquid pre-concentrates. At each time point the content of the vial was diluted 1 to 20 with Mestex buffer without the surfactants. After 5 min, samples were taken, diluted 1:1 with ethanol and analyzed by HPLC. Moreover, a calibration curve was established at each time point using the RTX dilutions made from the stock solution in order to ensure comparability of the obtained data.

In table 3 below the results of RTX stability in such compositions are shown as percentage of the T0 value which was measured after an initial filtration and centrifugation.

**Table 3:**

| | Tween¹/EtOH | | Tween²/EtOH | | | Creph. EL/EtOH | | | Creph. RH60/EtOH | | | Creph. E/ Mannitol | | Lipoid S100 | | Lipoid S100/ Mannitol | |
|---|---|---|---|---|---|---|---|---|---|---|---|---|---|---|---|---|---|
| | 4°C | 25°C | 4°C | 25°C | 40°C | 4°C | 25°C | 40°C | 4°C | 25°C | 40°C | 4°C | 25°C | 4°C | 25°C | 4°C | 25°C |
| T0 | 95.0 | 95.0 | 100.0 | 100.0 | 100.0 | 100.0 | 100.0 | 100.0 | 100.0 | 100.0 | 100.0 | | 102.0 | | 66.0 | | 70.0 |
| 1 m | 97.3 | 92.8 | | | | | | | | | | | | | | | |
| 2 m | 93.4 | 77.1 | | | | | | | | | | | | | | | |
| 3 m | 94.3 | 77.7 | 100.0 | 100.0 | 90.0 | 100.0 | 100.0 | 90.0 | 100.0 | 100.0 | 88.0 | | | | | | |
| 6 m | | | 100.0 | 100.0 | | 100.0 | 100.0 | | 100.0 | 100.0 | | | | | | | |
| 9 m | | | | | | | | | | | | | | | | | |
| 12 m | | | 100.0 | 100.0 | | 95.0 | 100.0 | | 100.0 | 100.0 | | | | | | | |

| | | | | | | | | | | | | | | | | | |
|---|---|---|---|---|---|---|---|---|---|---|---|---|---|---|---|---|---|
| ¹= TWEEN 14%; ²=Tween 0.08% | | | | | | | | | | | | | | | | | |

Table 3 shows stability data of non-aqueous liquid compositions. All reconstituted solutions were clear and colorless.

All the non-aqueous liquid compositions still contain 5% solvent and together with the fact that for such formulations an extra vial with the corresponding buffer has to be prepared and delivered, the use of non-aqueous liquid formulations is less preferred compared to the non-aqueous solid compositions described in here.

### Example 3:

### Rehydration of non-aqueous solid RTX compositions and short term stability

An appropriate amount of water for injection (WFI) was added to a vial containing the lyophilized RTX composition as prepared in Example 1 comprising TPGS/Mestex Buffer ("TPGS/MB" as designated in Table 2 above at three different concentrations 0.1 %, 0.35 % and 0.7 %) to prepare the ready to use medication for intra-articular injection into joints. As can be seen from Table4 below, the rehydrated lyophilized RTX compositions are stable over the whole measured period of four hours independent of the TPGS concentrations used.

**Table 4:**

| | TPGS concentration in final mixture | | | | | |
|---|---|---|---|---|---|---|
| | 0.10% | | 0.35% | | 0.70% | |
| | 4°C | 25°C | 4°C | 25°C | 4°C | 25°C |
| T0 | 100±2.1 | | 100±5.8 | | 100.0±2.6 | |
| 1h | 101.9 ±2.2 | 101.5±3.7 | 107.1±2.7 | 101.4±1.5 | 98.3±2.8 | 96.8±7.7 |
| 2h | 102.7 ±5.3 | 100.2±3.7 | 105.2±1.9 | 101.0±2.5 | 100.4±4.9 | 99.3±8.5 |
| 4h | 101.9±3.3 | 100.1±1.9 | 103.5±1.2 | 101.9±2.2 | 98.6±6.0 | 96.9±7.2 |

Table 4 shows results of stability studies of the formulation RTX/EtOH/0.10, 0.35 or 0.70 % TPGS/Mestex buffer. Samples were diluted 1:1 with EtOH before HPLC analysis. Indicated values are means (n ≥ 3) ± SD.

### ITEM LIST

Amongst others, the present invention relates to the following specific embodiments:
1. A non-aqueous composition of resiniferatoxin, the composition comprising: (a) resiniferatoxin; and (b) a surfactant.
2. The composition of item 1, wherein the composition is a solid composition.
3. The composition of item 2, wherein the solid composition is a lyophilized composition.
4. The composition of any one of items 2 to 3, wherein the surfactant is a non-ionic surfactant.
5. The composition of item 4, wherein the surfactant is selected from the group consisting of D-α-tocopherol polyethylene glycol succinate, PEG-20 hydrogenated castor oil, PEG-35 hydrogenated castor oil, PEG-40 hydrogenated castor oil, PEG-60 hydrogenated castor oil, PEG-100 hydrogenated castor oil, PEG-1000 hydrogenated castor oil, PEG-2000 hydrogenated castor oil, PEG-4000 hydrogenated castor oil, PEG-6000 hydrogenated castor oil, polysorbate 20, polysorbate 40, polysorbate 60, polysorbate 80, and polysorbate 100.
6. The composition of item 5, wherein the surfactant is selected from the group consisting of D-α-tocopherol polyethylene glycol succinate, PEG-60 hydrogenated castor oil, PEG-40 hydrogenated castor oil, PEG-35 hydrogenated castor oil, and polysorbate 80.
7. The composition of item 6, wherein the surfactant is D-α-tocopherol polyethylene glycol succinate.
8. The composition of item 6, wherein the surfactant is PEG-60 hydrogenated castor oil.
9. The composition of item 6, wherein the surfactant is PEG-40 hydrogenated castor oil.
10. The composition of item 6, wherein the surfactant is PEG-35 hydrogenated castor oil.
11. The composition of item 6, wherein the surfactant is polysorbate 80.
12. The composition of any one of items 2 to 11, further comprising: (d) a polyethylene glycol and/or (e) a further surfactant, wherein said further surfactant is optionally a poloxamer.
13. The composition of item 12, wherein the polyethylene glycol is PEG 1000, PEG 1450, PEG 1600, PEG 3000, PEG 3350, PEG 4000, PEG 4500, PEG 6000, PEG 8000, or PEG 20000.
14. The composition of item 13, wherein the polyethylene glycol is PEG 6000.
15. The composition of any one of items 2 to 14, wherein at least 90 % of the resiniferatoxin is recovered after dissolution of the solid in an aqueous diluent.
16. The composition of item 15, wherein at least 95 % of the resiniferatoxin is recovered after dissolution of the solid in an aqueous diluent.
17. The composition of any one of items 2 to 16, wherein at least 90 % of the resiniferatoxin is recovered after storage at about 0 °C to about 25 °C for up to about 3 to about 24 months.
18. The composition of item 17, wherein at least 95 % of the resiniferatoxin is recovered after storage at 0 °C to 25 °C for up to about 3 to about 12 months.
19. The composition of any one of items 2 to 18, wherein the composition further comprises NaCl.
20. The composition of any one of items 2 to 19, wherein the composition further comprises CaCl₂ x 2 H₂O.
21. The composition of any one of items 2 to 20, wherein the composition further comprises KCl.
22. The composition of any one of items 2 to 21, wherein the composition further comprises *tris*(hydroxymethyl)aminomethane.
23. The composition of any one of items 2 to 7 and 12 to 22 wherein the composition comprises resiniferatoxin, D-α-tocopherol polyethylene glycol succinate, NaCl, CaCl₂ x 2 H₂O, KCl, and *tris*(hydroxymethyl)aminomethane.
24. The composition of any one of items 2 to 23 for use in the manufacture of a medicament for the treatment of pain.
25. The composition of item 24, wherein the pain is osteoarthritis-related joint pain.
26. A kit comprising: (a) a first kit component comprising the composition of any one of items 2 to 25; and (b) a second kit component comprising a diluent.
27. The kit of item 26, wherein the diluent is an aqueous diluent.
28. The kit of item 27, wherein the aqueous diluent comprises at least one buffering agent and optionally at least one isotonizing agent.
29. The kit of item 28, wherein the at least one buffering agent is adapted to maintain a pH from about 7.7 to about 8.3 upon combining the first and the second kit components.
30. The kit of item 29, wherein the aqueous diluent comprises about 140 - 150 mM NaCl, about 3 - 5 mM CaCl₂ x 2 H₂O, about 3 - 5 mM KCl, about 8 - 12 mM *tris*(hydroxymethyl)aminomethane, and has a pH from about 7.7 to about 8.3.
31. The kit of item 30, wherein the aqueous diluent comprises about 147 mM NaCl, about 4 mM CaCl₂ x 2 H₂O, about 4 mM KCl, about 10 mM *tris*(hydroxymethyl)aminomethane, and has a pH of about 8.0.
32. A method of preparing a non-aqueous solid composition of resiniferatoxin, the method comprising: (a) combining resiniferatoxin and a surfactant in a solvent; (b) removing the solvent.
33. The method of item 32, wherein a buffer is combined with the resiniferatoxin and a surfactant in a solvent in step (a).
34. The method of any one of items 32 to 33, wherein the solvent is removed by lyophilization.
35. A non-aqueous solid composition of resiniferatoxin, prepared according to the method of any one of items 32 to 34.
36. The composition of item 1, wherein the composition is a liquid composition.
37. The composition of item 36, wherein the concentration of resiniferatoxin is about from about 0.005 µM to about 10 µM resiniferatoxin, preferably from about 0.01 µM to about 1 µM resiniferatoxin, even more preferably from about 0.1 µM to about 0.2 µM resiniferatoxin; (b) from about 0.01 vol % to about 10 vol % of a surfactant, preferably from about 0.01 vol % to about 1 vol % of a surfactant, even more preferably about 0.1 vol % to about 0.5 vol % of a surfactant, in particular 0.35 vol % of a surfactant.
38. The composition of item 36 or 37, wherein the surfactant is a non-ionic surfactant.
39. The composition of item 38, wherein the surfactant is selected from the group consisting of D-α-tocopherol polyethylene glycol succinate, PEG-20 hydrogenated castor oil, PEG-35 hydrogenated castor oil, PEG-40 hydrogenated castor oil, PEG-60 hydrogenated castor oil, PEG-100 hydrogenated castor oil, PEG-1000 hydrogenated castor oil, PEG-2000 hydrogenated castor oil, PEG-4000 hydrogenated castor oil, PEG-6000 hydrogenated castor oil, polysorbate 20, polysorbate 40, polysorbate 60, polysorbate 80, and polysorbate 100.
40. The composition of item 39, wherein the surfactant is selected from the group consisting of D-α-tocopherol polyethylene glycol succinate, PEG-60 hydrogenated castor oil, PEG-40 hydrogenated castor oil, PEG-35 hydrogenated castor oil, and polysorbate 80.
41. The composition of item 40, wherein the surfactant is D-α-tocopherol polyethylene glycol succinate.
42. The composition of item 40, wherein the surfactant is PEG-60 hydrogenated castor oil.
43. The composition of item 40, wherein the surfactant is PEG-40 hydrogenated castor oil.
44. The composition of item 40, wherein the surfactant is PEG-35 hydrogenated castor oil.
45. The composition of item 40, wherein the surfactant is polysorbate 80.
46. The composition of any one of items 36 to 45, further comprising: (d) a polyethylene glycol; and/or (e) a further surfactant, wherein the further surfactant is optionally a poloxamer.
47. The composition of item 46, wherein the polyethylene glycol is PEG 1000, PEG 1450, PEG 1600, PEG 3000, PEG 3350, PEG 4000, PEG 4500, PEG 6000, PEG 8000, and PEG 20000.
48. The composition of item 47, wherein the polyethylene glycol is PEG 6000.
49. The composition of any one of items 36 to 48, wherein at least 90 % of the resiniferatoxin is recovered after storage at about 0 °C to about 25 °C for up to about 3 to about 24 months.
50. The composition of item 49, wherein at least 95 % of the resiniferatoxin is recovered after storage at about 0 °C to about 25 °C for up to about 3 to about 12 months.
51. The composition of any one of items 36 to 50 for use in the manufacture of a medicament for the treatment of pain.
52. The composition of item 51, wherein the pain is osteoarthritis-related joint pain.
53. A kit comprising: (a) a first kit component comprising the composition of any one of items 36 to 52; and (b) a second kit component comprising a diluent.
54. The kit of item 53, wherein the diluent is an aqueous diluent.
55. The kit of item 54, wherein the aqueous diluent comprises at least one buffering agent and optionally at least one isotonizing agent.
56. The kit of item 55, wherein the at least one buffering agent is adapted to maintain a pH from about 7.7 to about 8.3 upon combining the first and the second kit components.
57. The kit of item 56, wherein the aqueous diluent comprises about 140 - 150 mM NaCl, about 3 - 5 mM CaCl₂ x 2 H₂O, about 3 - 5 mM KCl, about 8 - 12 mM *tris*(hydroxymethyl)aminomethane, and has a pH from about 7.7 to about 8.3.
58. The kit of item 57, wherein the aqueous buffer diluent comprises about 147 mM NaCl, about 4 mM CaCl₂ x 2 H₂O, about 4 mM KCl, about 10 mM *tris*(hydroxymethyl)aminomethane, and has a pH of about 8.0.
59. A method of preparing a non-aqueous liquid composition of resiniferatoxin, the method comprising combining resiniferatoxin, a surfactant and a non-aqueous solvent.
60. A non-aqueous liquid composition of resiniferatoxin prepared according to the method of item 59.
61. An aqueous composition of resiniferatoxin, the composition comprising: (a) from about 0.005 µM to about 10 µM resiniferatoxin, preferably from about 0.01 µM to about 1 µM resiniferatoxin, more preferably from about 0.1 µM to about 1 µM resiniferatoxin, even more preferably from about 0.1 µM to about 0.2 µM resiniferatoxin; (b) from about 0.01vol % to about 10 vol % of a surfactant, preferably from about 0.01 vol % to about 1 vol % of a surfactant, more preferably from about 0.1 vol % to about 1 vol % of a surfactant, even more preferably about 0.1 vol % to about 0.5 vol % of a surfactant, in particular 0.35 vol % of a surfactant; and (c) at least one buffering agent; wherein the composition has a pH from about 7.7 to about 8.3.
62. The composition of item 61, comprising about 140 - 150 mM NaCl, about 3 - 5 mM CaCl₂ x 2 H₂O, about 3-5 mM KCl, about 8 - 12 mM *tris*(hydroxymethyl)aminomethane.
63. The composition of item 62, comprising about 147 mM NaCl, about 4 mM CaCl₂ x 2 H₂O, about 4 mM KCl, about 10 mM *tris*(hydroxymethyl)aminomethane.
64. The composition of any one of item 61 to 63, wherein the composition is adapted for intra-articular administration.
65. The composition of any one of item 61 to 64, wherein the composition is obtainable by combining the first and the second kit components as defined in any one of item 26 to 31 or item 53 to 58.
66. The composition of any one of items 61 to 65 for use in the treatment of pain.
67. The composition of item 66, wherein the pain is osteoarthritis-related joint pain.
68. A method of treating pain, comprising administering to a subject a therapeutically effective amount of the composition of any one of items 61 to 65.
69. The method of item 68, wherein the pain is osteoarthritis-related joint pain.
70. The composition of any one of items 1 to 25, 35 to 52, and 60 to 67, wherein the composition does not comprise a monosaccharide or sugar alcohol.
71. A dilution of the composition of any one of items 1 to 6 comprising: (a) from about 0.005 µM to about 10 µM resiniferatoxin preferably from about 0.01 µM to about 1 µM resiniferatoxin, more preferably from about 0.1 µM to about 1 µM resiniferatoxin, even more preferably from about 0.1 µM to about 0.2 µM resiniferatoxin; (b) from about 0.01vol % to about 10 vol % of a surfactant, preferably from about 0.01 vol % to about 1 vol % of a surfactant, more preferably from about 0.1 vol % to about 1 vol % of a surfactant, even more preferably about 0.1 vol % to about 0.5 vol % of a surfactant, in particular 0.35 vol % of a surfactant; and
   (c) at least one buffering agent; wherein the composition has a pH from about 7.7 to about 8.3.
72. The dilution of the composition of item 71, comprising about 140 - 150 mM NaCl, about 3 - 5 mM CaCl₂ x 2 H2O, about 3 - 5 mM KCl, about 8 - 12 mM tris(hydroxymethyl)aminomethane.
73. The dilution of the composition of item 72, comprising about 147 mM NaCl, about 4 mM CaCl₂ x 2 H₂O, about 4 mM KCl, about 10 mM *tris*(hydroxymethyl)aminomethane.
74. The dilution of the composition of any one of item 71 to 73, wherein the composition is adapted for intra-articular administration.
75. The dilution of the composition of any one of item 71 to 74, wherein the composition is obtainable by combining the first and the second kit components as defined in any one of item 26 to 31 or item 53 to 58.
76. The dilution of the composition of any one of items 71 to 75 for use in the treatment of pain.
77. The dilution of the composition of item 76, wherein the pain is osteoarthritis-related joint pain.
78. A method of treating pain, comprising administering to a subject a therapeutically effective amount of the dilution of the composition of any one of items 71 to 75.
79. The method of item 78, wherein the pain is osteoarthritis-related joint pain.
80. The dilution of the composition of any one of items 1 to 25, 35 to 52, and 71 to 77, wherein the composition does not comprise a monosaccharide or sugar alcohol.

### CLAUSES LIST

The present invention further relates to the following specific embodiments:

| | |
|---|---|
| Clause 1: | A non-aqueous composition of resiniferatoxin, the composition comprising: (a) resiniferatoxin; and (b) a surfactant, wherein the surfactant is D-α-tocopherol polyethylene glycol succinate. |
| Clause 2: | The composition of clause 1, wherein the composition is a solid composition. |
| Clause 3: | The composition of clause 2, wherein the solid composition is a lyophilized composition. |
| Clause 4: | The composition of any one of clauses 1 to 3, further comprising: (d) a polyethylene glycol and/or (e) a further surfactant, wherein said further surfactant is optionally a poloxamer. |
| Clause 5: | The composition of any one of clauses 1 to 4, wherein at least 90 % of the resiniferatoxin is recovered after dissolution of the solid in an aqueous diluent. |
| Clause 6: | The composition of any one of clauses 1 to 5, wherein the composition comprises resiniferatoxin, D-α-tocopherol polyethylene glycol succinate, NaCl, CaCl₂ x 2 H₂O, KCl, and *tris*(hydroxymethyl)aminomethane. |
| Clause 7: | A kit comprising: (a) a first kit component comprising the composition of any one of clauses 2 to 6; and (b) a second kit component comprising a diluent. |
| Clause 8: | The composition of clause 1, wherein the composition is a liquid composition. |
| Clause 9: | The composition of clause 8, wherein the concentration of resiniferatoxin is about 0.005 µM to about 10 µM and the concentration of the surfactant is about 0.01 vol % to about 10 vol %. |
| Clause 10: | A kit comprising: (a) a first kit component comprising the composition of any one of clauses 8 to 9; and (b) a second kit component comprising a diluent. |
| Clause 11: | An aqueous composition of resiniferatoxin, the composition comprising: (a) from about 0.005 µM to about 10 µM resiniferatoxin; (b) from about 0.01 vol % to about 10 vol % of a surfactant; and (c) at least one buffering agent; wherein the composition |
| | has a pH from about 7.7 to about 8.3 and wherein the surfactant is D-α-tocopherol polyethylene glycol succinate. |
| Clause 12: | The composition of any one of clauses 1 to 11 for use in a method for the treatment of pain. |
| Clause 13: | The composition of any one of clauses 1 to 11, wherein the composition does not comprise a monosaccharide or sugar alcohol. |

## Claims

1. A solid non-aqueous composition of resiniferatoxin, preferably for use in the treatment of osteoarthritis-related joint pain, the composition comprising:
(a) resiniferatoxin; and
(b) a non-ionic surfactant selected from the group consisting of D-α-tocopherol polyethylene glycol succinate, PEG-20 hydrogenated castor oil, PEG-35 hydrogenated castor oil, PEG-40 hydrogenated castor oil, PEG-60 hydrogenated castor oil, PEG-100 hydrogenated castor oil, PEG-1000 hydrogenated castor oil, PEG-2000 hydrogenated castor oil, PEG-4000 hydrogenated castor oil, PEG-6000 hydrogenated castor oil, polysorbate 20, polysorbate 40, polysorbate 60, polysorbate 80, and polysorbate 100.

2. The composition according to claim 1, wherein the composition further comprises NaCl.

3. The composition according to claim 1 or 2, wherein the composition further comprises CaCl₂ x 2 H₂O.

4. The composition according to any of the preceding claims, wherein the composition further comprises KCl.

5. The composition according to any of the preceding claims, wherein the composition further comprises *tris*(hydroxymethyl)aminomethane.

6. The composition according to any of the preceding claims, wherein the composition comprises resiniferatoxin, D-α-tocopherol polyethylene glycol succinate, NaCl, CaCl₂ x 2 H₂O, KCl, and *tris*(hydroxymethyl)aminomethane.

7. The composition according to any of the preceding claims for use in the treatment of pain; preferably wherein the pain is osteoarthritis-related joint pain.

8. A kit comprising:
(a) a first kit component comprising the composition according to any of the preceding claims; and
(b) a second kit component comprising a diluent.

9. The kit according to claim 8, wherein the diluent is an aqueous diluent; preferably wherein the aqueous diluent comprises at least one buffering agent and optionally at least one isotonizing agent; preferably wherein the at least one buffering agent is adapted to maintain a pH from about 7.7 to about 8.3 upon combining the first and the second kit components.

10. The kit according to claim 9, wherein the aqueous diluent comprises about 140 - 150 mM NaCl, about 3 - 5 mM CaCl₂ x 2 H₂O, about 3 - 5 mM KCl, about 8 - 12 mM *tris*(hydroxymethyl)aminomethane, and has a pH from about 7.7 to about 8.3; preferably about 147 mM NaCl, about 4 mM CaCl₂ x 2 H₂O, about 4 mM KCl, about 10 mM *tris*(hydroxymethyl)aminomethane, and has a pH of about 8.0.

11. An aqueous composition of resiniferatoxin, preferably for use in the treatment of osteoarthritis-related joint pain, the composition comprising:
(a) from about 0.005 µM to about 10 µM resiniferatoxin, preferably from about 0.01 µM to about 1 µM resiniferatoxin, more preferably from about 0.1 µM to about 1 µM resiniferatoxin, even more preferably from about 0.1 µM to about 0.2 µM resiniferatoxin;
(b) from about 0.01vol % to about 10 vol % of a surfactant, preferably from about 0.01 vol % to about 1 vol % of a surfactant, more preferably from about 0.1 vol % to about 1 vol % of a surfactant, even more preferably about 0.1 vol % to about 0.5 vol % of a surfactant, in particular 0.35 vol % of a surfactant;
wherein the surfactant is a non-ionic surfactant selected from the group consisting of D-α-tocopherol polyethylene glycol succinate, PEG-20 hydrogenated castor oil, PEG-35 hydrogenated castor oil, PEG-40 hydrogenated castor oil, PEG-60 hydrogenated castor oil, PEG-100 hydrogenated castor oil, PEG-1000 hydrogenated castor oil, PEG-2000 hydrogenated castor oil, PEG-4000 hydrogenated castor oil, PEG-6000 hydrogenated castor oil, polysorbate 20, polysorbate 40, polysorbate 60, polysorbate 80, and polysorbate 100; and
(c) at least one buffering agent; wherein the composition has a pH from about 7.7 to about 8.3.

12. The composition according to claim 11, comprising about 140 - 150 mM NaCl, about 3 - 5 mM CaCl₂ x 2 H₂O, about 3 - 5 mM KCl, about 8 - 12 mM *tris*(hydroxymethyl)-aminomethane; preferably about 147 mM NaCl, about 4 mM CaCl₂ x 2 H₂O, about 4 mM KCl, about 10 mM *tris*(hydroxymethyl)aminomethane.

13. The composition according to claim 11 or 12, wherein the composition is adapted for intra-articular administration.

14. The composition according to any of claims 11 to 13, wherein the composition is obtainable by combining the first and the second kit components as defined in any one of claims 8 to 10.

15. The composition according to any of claims 11 to 14 for use in the treatment of pain; preferably wherein the pain is osteoarthritis-related joint pain.
